(19) Europäisches Patentamt
European Patent Office
Office européen des brevets

(11) EP 4 391 582 A1

(12) **EUROPEAN PATENT APPLICATION**
published in accordance with Art. 153(4) EPC

(43) Date of publication:
26.06.2024 Bulletin 2024/26

(21) Application number: 22882408.2

(22) Date of filing: 11.08.2022

(51) International Patent Classification (IPC):
*H04R 1/10* (2006.01)

(52) Cooperative Patent Classification (CPC):
G01K 13/20; H04R 1/10

(86) International application number:
PCT/CN2022/111679

(87) International publication number:
WO 2023/065787 (27.04.2023 Gazette 2023/17)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 22.10.2021 CN 202111233939

(71) Applicant: Huawei Technologies Co., Ltd.
Shenzhen, Guangdong 518129 (CN)

(72) Inventors:
• ZHANG, Yuchao
  Shenzhen, Guangdong 518129 (CN)
• LU, Lin
  Shenzhen, Guangdong 518129 (CN)

(74) Representative: Pfenning, Meinig & Partner mbB
Patent- und Rechtsanwälte
Theresienhöhe 11a
80339 München (DE)

(54) **EARPHONES AND TEMPERATURE MEASUREMENT METHOD OF EARPHONES**

(57) This application provides an earphone and a temperature measurement method for the earphone. The earphone may include an earbud and a first temperature sensor. The earbud includes an earbud housing and a sound outlet hole, first accommodating space is formed by enclosing the earbud housing, the sound outlet hole may be disposed on the earbud housing, and the sound outlet hole connects the first accommodating space and external space of the earphone, so that the earphone can emit sound through the sound outlet hole. Therefore, in this application, the first temperature sensor may be located in the first accommodating space, and may be disposed on an outer side of a hole channel of the sound outlet hole, to avoid impact of the first temperature sensor on sound outlet effect of the sound outlet hole. In addition, the sound outlet hole may be disposed directly opposite to a periosteum of a human ear. Therefore, a temperature of the periosteum can be transferred to the sound outlet hole. In addition, the temperature of the periosteum of the human ear is close to a core temperature of a human body. Based on this, the first temperature sensor can be in thermally conductive contact with the sound outlet hole, so that the temperature of the periosteum can be transferred to the first temperature sensor through the sound outlet hole, and the first temperature sensor can obtain a more accurate human body temperature through measurement.

FIG. 3

**Description**

**CROSS-REFERENCE TO RELATED APPLICATIONS**

This application claims priority to Chinese Patent Application No. 202111233939.9, filed with the China National Intellectual Property Administration on October 22, 2021 and entitled "EARPHONE AND TEMPERATURE MEASUREMENT METHOD FOR EARPHONE", which is incorporated herein by reference in its entirety.

**TECHNICAL FIELD**

This application relates to the field of wearable electronic device technologies, and in particular, to an earphone and a temperature measurement method for the earphone.

**BACKGROUND**

Nowadays, people pay more and more attention to health statuses of themselves and their families, and a body temperature is an important index for representing a health status of a human body. In addition, because body temperature measurement is convenient, currently, increasingly more daily used electronic devices, especially some intelligent wearable devices, are provided with body temperature measurement modules, to meet a requirement of a user for body temperature measurement at any time.

Currently, most electronic devices with body temperature measurement modules adopt a contact temperature measurement solution. To improve temperature measurement accuracy of the contact temperature measurement solution, the body temperature measurement module usually needs to be in full contact with a skin surface to be measured, so that the body temperature measurement module can fully sense a body temperature of the user. In addition, in order to fully contact the body temperature measurement module with the skin surface to be measured, the body temperature measurement module needs to be provided with a regular measurement part, and the skin surface to be measured also needs to be as flat and regular as possible. However, not all surfaces of the human body have features of flatness and regularity, for example, a skin surface of a human ear is rough and uneven. Therefore, it is difficult for the body temperature measurement module disposed in the electronic device (for example, an earphone) worn on the human ear to be in full contact with human ear skin. Consequently, body temperature measurement accuracy cannot be ensured.

Based on this, how to dispose the body temperature measurement module in the electronic device worn on the human ear, like the earphone, to obtain a more accurate body temperature measurement result by measuring a human ear temperature has become a technical problem to be urgently resolved by a person skilled in the art.

**SUMMARY**

This application provides an earphone and a temperature measurement method for the earphone, to improve body temperature measurement accuracy of a body temperature measurement module by properly designing the body temperature measurement module in the earphone.

According to a first aspect, an earphone provided in this application may include an earbud and a first temperature sensor. When the earbud is specifically disposed, the earbud may include an earbud housing and a sound outlet hole. The earbud housing has first accommodating space, and at least a part of a structure of the earbud may be disposed in the first accommodating space. The sound outlet hole is disposed on the earbud housing, and the sound outlet hole may be configured to connect the first accommodating space and external space of the earphone, so that the earphone can emit sound through the sound outlet hole. The first temperature sensor is located in the first accommodating space, and the first temperature sensor is disposed on an outer side of a hole channel of the sound outlet hole, to avoid blocking the sound outlet hole, and impact of the first temperature sensor on sound outlet effect of the sound outlet hole is slight. In addition, when the earphone is worn on a human ear, the sound outlet hole may be usually disposed opposite to a periosteum of the human ear, and a temperature of the periosteum is close to a temperature of a core part of a human body. Therefore, in this application, the first temperature sensor can be in thermally conductive contact with the sound outlet hole through a sound outlet net. In this way, the temperature of the periosteum can be transferred to the sound outlet hole through air, and then transferred to the first temperature sensor through the sound outlet net, so that the first temperature sensor can obtain a more accurate human body temperature through measurement.

To effectively transfer the temperature at the sound outlet hole to the first temperature sensor, in this application, when the sound outlet net is specifically disposed, at least a part of the sound outlet net may be provided with a thermally conductive path. The thermally conductive path may be made of, but is not limited to, a high thermally conductive material such as copper, stainless steel, nickel, titanium, gold, or silver. In this way, the first temperature sensor can be in thermally conductive contact with the thermally conductive path. After the temperature of the periosteum is transferred to the sound outlet hole, the temperature can be transferred to the first temperature sensor through the thermally conductive path. This helps improve accuracy of temperature data collected by the first temperature sensor.

In a possible implementation of this application, the earphone may include a circuit board. The circuit board may be disposed on the outer side of the hole channel of the sound outlet hole, and the circuit board is in thermally conductive contact with the sound outlet hole. In addition, the first temperature sensor is electrically connected to the circuit board. Because a large quantity of metal wires are usually disposed on the circuit board, the temperature at the sound outlet hole may be transferred to the first temperature sensor by using the circuit board.

In some possible embodiments of this application, in addition to the first temperature sensor, the earphone may further include a second temperature sensor. The second temperature sensor may be located in the first accommodating space, and the second temperature sensor may be fastened to an inner side wall corresponding to a position at which the earbud housing is in contact with human ear skin. In this way, the second temperature sensor can perform measurement on the human ear skin, the human body temperature can be obtained based on a mapping relationship between the temperature data collected by the first temperature sensor and temperature data collected by the second temperature sensor, to correct the temperature data collected by the first temperature sensor. This can reduce impact of a human ear skin temperature on human body temperature measurement, and improve accuracy of human body temperature measurement.

In addition, to improve thermally conductive efficiency between the human ear skin and the second temperature sensor, the earbud housing may be provided with a first thermally conductive portion, the first thermally conductive portion may be made of a metal material, and the first thermally conductive portion is in contact with the human ear skin. Because thermal conductivity of the metal material is high, the second temperature sensor may be in thermally conductive contact with the first thermally conductive portion, so that the human ear skin temperature is transferred to the second temperature sensor through the first thermally conductive portion. This helps improve accuracy of the temperature data collected by the second temperature sensor.

In this application, the earphone may further include an earphone handle, and the earphone handle may be fastened to the earbud. The earphone handle may include an earphone handle housing, the earphone handle has second accommodating space, and the second accommodating space may be connected to the first accommodating space. This implements connection between a structure in the earphone handle and a structure in the earbud.

In a possible implementation of this application, the earphone may further include a third temperature sensor, and the third temperature sensor may be located in the second accommodating space. In addition, when it is considered that a most part of a structure of the earphone handle housing may be in contact with the external space, the third temperature sensor may be fastened to an inner side wall corresponding to a position at which the earphone handle housing is in contact with the external space, so that the third temperature sensor can be configured to collect a temperature of the external space. In this way, the temperature data collected by the first temperature sensor can be corrected based on both the temperature data collected by the second temperature sensor and the temperature data collected by the third temperature sensor, to reduce impact of the human ear skin temperature and the temperature of the external space on human body temperature measurement, and obtain the human body temperature. This helps improve accuracy of human body temperature measurement.

In addition, to improve thermally conductive efficiency between the external space and the third temperature sensor, the earbud housing may be provided with a second thermally conductive portion, the second thermally conductive portion may be made of a metal material, and the second thermally conductive portion is in contact with the external space. Because the thermal conductivity of the metal material is high, the third temperature sensor may be in thermally conductive contact with the second thermally conductive portion, so that the temperature of the external space can be transferred to the third temperature sensor through the second thermally conductive portion. This helps improve accuracy of the temperature data collected by the third temperature sensor.

A heat emitting element is usually disposed in the earphone. The heat emitting element may be disposed in the first accommodating space and/or the second accommodating space. In a working process, a temperature of the heat emitting element gradually increases, and it is difficult to diffuse the temperature to the outside of the earphone in time, and the temperature may converge inside the earphone. To reduce impact of heat generated by the heat emitting element on the temperature measured by the third temperature sensor, in a possible implementation of this application, a fourth temperature sensor may be disposed in the earphone. The fourth temperature sensor may be disposed in the first accommodating space and/or the second accommodating space, and the fourth temperature sensor is disposed close to the heat emitting element. For example, the fourth temperature sensor and the heat emitting element may be separately disposed on two sides of a same circuit board, and the fourth temperature sensor is disposed facing away from the heat emitting element. In this way, the fourth temperature sensor can obtain a more accurate temperature of the heat emitting element.

In addition, in a possible implementation of this application, the fourth temperature sensor and the third temperature sensor may be alternatively spaced from each other. Therefore, a temperature gradient exists between positions at which the third temperature sensor and the fourth temperature sensor are located, and the temperature data collected by the third temperature sensor is corrected based on the temperature gradient, to obtain a more accurate ambient temperature. In this way, the temperature collected by the first temperature sensor can be corrected based on the ambient temperature and the temperature data collected by the second temperature sensor, to reduce impact of the human ear skin temperature, the ambient temperature, and the temperature of the heat emitting element on human body temperature measurement. This can effectively improve body temperature measurement precision.

In a possible implementation of this application, the earphone may further include a speaker, and the speaker is accommodated in the first accommodating space. The speaker may include a first sound emitting unit and a second sound emitting unit, and the first sound emitting unit and the second sound emitting unit each may be responsible for sound emitting in a different frequency band. When the first sound emitting unit is specifically disposed, the first sound emitting unit includes a first diaphragm and a coil disposed on the first diaphragm. In addition, when the second sound emitting unit is specifically disposed, the second sound emitting unit may include a second diaphragm and a magnetic circuit component. In this application, the second sound emitting unit may be disposed between the first sound emitting unit and the sound outlet hole. When the speaker works, the coil of the first sound emitting unit may drive the first diaphragm to vibrate under driving of a signal current. When the first diaphragm vibrates, the first diaphragm may push air disturbance in space between the first diaphragm and the sound outlet hole, and produce resonance with air in the space to emit sound. Alternatively, the magnetic circuit component of the second sound emitting unit drives the second diaphragm to vibrate. When the second diaphragm vibrates, the second diaphragm may push air disturbance in space between the second diaphragm and the sound outlet hole, and produce resonance with air in the space to emit sound. The sound may be transmitted from the inside of the earbud to the outside of the earbud through the hole channel of the sound outlet hole, so that the sound is spread in an ear canal of the human ear. Finally, the sound is emitted by the earphone.

According to a second aspect, this application further provides a temperature measurement method for an earphone. The earphone may include an earbud and a first temperature sensor, the earbud may include an earbud housing and a sound outlet hole, the earbud housing has first accommodating space, the sound outlet hole is disposed on the earbud housing, and the sound outlet hole connects the first accommodating space and external space of the earphone. The first temperature sensor is located in the first accommodating space, the first temperature sensor is disposed on an outer side of a hole channel of the sound outlet hole, and the first temperature sensor is in thermally conductive contact with the sound outlet hole. The temperature measurement method may include:

performing wear detection on the earphone, and determining a wearing status of the earphone on a human ear;
triggering a temperature measurement procedure of the earphone based on the wearing status of the earphone on the human ear, and controlling the first temperature sensor to start to collect temperature data; and
processing the temperature data collected by the first temperature sensor to obtain a human body temperature.

When the earphone is worn on the human ear, the sound outlet hole may be usually disposed opposite to a periosteum of the human ear, and a temperature of the periosteum is close to a temperature of a core part of a human body. Therefore, in this application, the first temperature sensor is in thermally conductive contact with the sound outlet hole through a sound outlet net, so that the temperature of the periosteum can be transferred to the sound outlet hole through air, and then transferred to the first temperature sensor through the sound outlet net. According to the temperature measurement method for the earphone provided in this application, when the earphone is effectively worn on the human ear, the first temperature sensor may start to collect the temperature data, and the temperature data collected by the first temperature sensor is processed, to obtain a more accurate human body temperature.

In this application, there are many methods for obtaining the human body temperature by processing the temperature data collected by the first temperature sensor. In a possible implementation, a temperature data curve may be formed based on the temperature data collected by the first temperature sensor, and the temperature data curve is processed to obtain the human body temperature.

In another possible implementation of this application, the earphone further includes a second temperature sensor, the second temperature sensor is configured to measure a human ear skin temperature, and the processing the temperature data collected by the first temperature sensor to obtain a human body temperature specifically includes:
obtaining the human body temperature based on the temperature data collected by the first temperature sensor and temperature data collected by the second temperature sensor.

In this way, the temperature data collected by the first temperature sensor can be corrected based on the temperature data collected by the second temperature sensor, to reduce impact of the human ear skin temperature on human body temperature measurement. This can improve accuracy of human body temperature measurement.

In addition, the earphone may further include a third temperature sensor, the third temperature sensor is configured to measure an ambient temperature of the external space of the earphone, and the temperature measurement method for the earphone may further include:
obtaining the human body temperature based on the temperature data collected by the first temperature sensor, the temperature data collected by the second temperature sensor, and temperature data collected by the third temperature sensor.

The first temperature sensor, the second temperature sensor, and the third temperature sensor are all disposed in the earphone, and the temperature data collected by the first temperature sensor can be corrected based on the temperature data collected by the second temperature sensor and the temperature data collected by the third temperature sensor, to reduce impact of the human ear skin temperature and the temperature of the external space on human body temperature measurement, and improve precision of human body temperature measurement.

In a possible implementation of this application, the earphone may further include a heat emitting element and a fourth temperature sensor, and the fourth temperature sensor is configured to measure a temperature of the heat emitting element. In this implementation, the temperature measurement method for the earphone may further include: obtaining the human body temperature based on the temperature data collected by the first temperature sensor, the temperature data collected by the second temperature sensor, the temperature data collected by the third temperature sensor, and temperature data collected by the fourth temperature sensor.

The first temperature sensor, the second temperature sensor, the third temperature sensor, and the fourth temperature sensor are all disposed in the earphone, and the temperature data collected by the first temperature sensor can be corrected based on the temperature data collected by the second temperature sensor, the temperature data collected by the third temperature sensor, and the temperature data collected by the fourth temperature sensor. During specific implementation, the temperature data collected by the third temperature sensor may be first corrected based on the temperature data collected by the fourth temperature sensor, and a corrected ambient temperature is obtained. Then, the human body temperature is obtained based on the temperature data collected by the first temperature sensor, the temperature data collected by the second temperature sensor, and the corrected ambient temperature. In this way, impact of the human ear skin temperature, the ambient temperature, and the temperature of the heat emitting element on human body temperature measurement can be effectively reduced, to improve precision of human body temperature measurement.

In a possible implementation of this application, the earphone may be connected to an external electronic device, and the external electronic device has a display interface. In this way, the method may further include: displaying the obtained human body temperature on the display interface. In this way, a user can conveniently view the measured human body temperature, so that the user can learn a health status of the user. This improves user experience.

## BRIEF DESCRIPTION OF DRAWINGS

FIG. 1 is a schematic diagram of a structure of an earphone according to an embodiment of this application;
FIG. 2 is a cross sectional view at A-A of the earphone in FIG. 1;
FIG. 3 is an enlarged view of a partial structure at B in FIG. 2;
FIG. 4 is a block diagram of a structure of an earphone according to an embodiment of this application;
FIG. 5 is a flowchart of a body temperature measurement method for an earphone according to an embodiment of this application;
FIG. 6 is a schematic diagram of a structure of an external electronic device according to an embodiment of this application;
FIG. 7a to FIG. 7c each are a schematic diagram of a trigger gesture in a temperature measurement process of an earphone according to an embodiment of this application;
FIG. 8 is a curve diagram of temperature data collected by a first temperature sensor according to an embodiment of this application;
FIG. 9 is a block diagram of a structure of an earphone according to another embodiment of this application;
FIG. 10 is a block diagram of a structure of an earphone according to another embodiment of this application;
FIG. 11 is a schematic diagram of a heat transfer path according to an embodiment of this application; and
FIG. 12 is a block diagram of a structure of an earphone according to another embodiment of this application.

Reference numerals:

1: earbud; 101: earbud housing; 1011: first accommodating space; 102: sound outlet hole; 1021: sound outlet net; 103: speaker; 1031: first sound emitting unit; 1032: second sound emitting unit; 2: earphone handle; 201: earphone handle housing;
2011: second accommodating space;

3: ear tip; 4: first temperature sensor; 5: circuit board; 6: external electronic device; 601: display interface; 7: second temperature sensor; 8: third temperature sensor; 9: heat emitting element; and 10: fourth temperature sensor.

## DESCRIPTION OF EMBODIMENTS

For ease of understanding an earphone provided in embodiments of this application, the following first describes an application scenario of the earphone. Key components such as an antenna, a processor chip, a battery, a microphone, a speaker, a wear sensor, and a control sensor may be integrated into the earphone provided in this application. The earphone may communicate with an electronic device, to implement interaction between the electronic device and the earphone. The electronic device herein includes but is not limited to terminal products such as a mobile phone, a tablet computer, a notebook computer, and a vehicle-mounted device, and wearable products such as a smart watch and a smart band. The earphone and the electronic device may be wiredly or wirelessly connected. A wireless connection manner between the earphone and the electronic device includes but is not limited to wireless connection by using a communication technology, for example, a wireless local area network (wireless local area networks, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, and an infrared (infrared, IR) technology.

In addition, from a perspective of a wearing manner, the earphone provided in embodiments of this application may be an in-ear earphone, or a semi-in-ear earphone. The in-ear earphone or the semi-in-ear earphone has advantages such as low sound leakage, good audio effect, and ease of carrying, and has been favored by consumers in recent years.

With rapid development of intelligent electronic devices, people also pose increasingly higher requirements on the intelligent electronic devices, to hope that the intelligent electronic devices can have more functions. In addition, currently, people pay more and more attention to health statuses of themselves and their families. To meet this requirement, health status monitoring functions are increasingly integrated into the intelligent electronic devices. Because a body temperature is an important index for representing a human body sign, body temperature measurement functions are increasingly integrated into the intelligent electronic devices.

Common intelligent electronic devices that have a body temperature measurement function include wearable devices such as a smart watch and a smart band. For example, a device such as a smart watch or a smart band may dispose a body temperature measurement module on a flat and regular structure on a side that is in contact with a human body, and skin of a wrist of the human body is flat. In this way, when the smart watch and the smart band are worn on the wrist of the human body, the body temperature measurement module can be in full contact with the skin of the wrist, and therefore it is convenient to measure the body temperature of the human body.

However, not all skin surfaces of the human body are flat and regular. For example, a skin surface of a human ear is rough and uneven, and there is no large flat surface that can be used for body temperature measurement. In addition, although a body temperature measurement module is currently integrated into some earphones, a user usually adjusts a wearing position and an angle of the earphone according to a personal preference and wearing comfort when wearing the earphone. In addition, it is difficult to ensure tightness of contact between the earphone and the human ear due to diversity of skin surface shapes of the human ear. Consequently, stability of body temperature measurement performed by the earphone by using the skin surface of the human ear cannot be ensured, and measurement precision of the earphone is low.

In the structure of the human ear, a temperature of a periosteum is closest to a core temperature of the human body. A sound outlet hole of the earphone is usually disposed directly opposite to the periosteum, so that sound is better transmitted to the human body through vibration of the periosteum. Based on this, currently, some earphones may select the sound outlet hole as a disposition position of the body temperature measurement module. However, space of the sound outlet hole is limited. If the body temperature measurement module is disposed at the sound outlet hole, a hole channel of the sound outlet hole becomes narrower. Consequently, audio effect of the earphone may be affected.

The earphone provided in this application is intended to resolve the foregoing problem, to transfer the body temperature to the body temperature measurement module inside the earphone through the sound outlet hole, to obtain a more accurate body temperature measurement result on the basis of not affecting audio effect of the sound outlet hole.

To make objectives, technical solutions, and advantages of this application clearer, the following further describes this application in detail with reference to the accompanying drawings and specific embodiments.

Terms used in the following embodiments are merely for the purpose of describing specific embodiments, but are not intended to limit this application. As used in the specification and appended claims of the application, singular expressions "one", "a", "the foregoing", "the", and "the one" are intended to further include expressions such as "one or more", unless otherwise specified in the context clearly. It should be further understood that in the following embodiments of this application, "at least one" and "one or more" refer to one, two, or more. The term "and/or" is used to describe an association relationship of associated objects and represents that three relationships may exist. For example, A and/or B may represent the following three cases: Only A exists, both A and B exist, and only B exists, where A and B may be singular or plural. The character "/" generally indicates an "or" relationship between the associated objects.

Reference to "an embodiment", "some embodiments", or the like described in this specification indicates that one or more embodiments of this application include a specific feature, structure, or characteristic described with reference to the embodiment. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

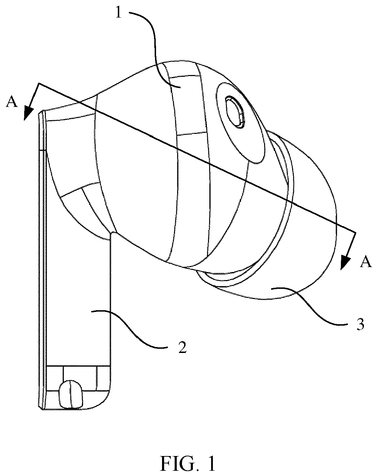

First, FIG. 1 is a schematic diagram of a structure of an earphone according to a possible embodiment of this application. In this embodiment, the earphone may include but is not limited to an earbud 1 and an earphone handle 2, and the earbud 1 is fastened to the earphone handle 2. In the embodiment shown in FIG. 1, an ear tip 3 may be disposed on the earbud 1, and the ear tip 3 is detachably connected to the earbud 1. A material of the ear tip 3 may be but is not limited to silica gel or rubber, that is, a material that can be deformed to some extent when being squeezed. When the earphone is worn on a human ear, the ear tip 3 may be inserted into an ear canal of the human ear, and squeezed with the ear canal, to implement reliable wearing of the earphone through an interference fit between the ear tip 3 and the ear canal. In some other possible embodiments of this application, a structure of the earbud 1 is properly designed, and the earbud 1 can be directly inserted into the ear canal, to implement wearing of the earphone through squeezing between the earbud 1 and the ear canal.

In this application, components such as a speaker and a wear sensor may be disposed in the earbud 1. This is convenient for the human ear to listen to audio data output by the earphone, and the wear sensor can detect a wearing status of the earphone on the human ear. Components such as a microphone, a control sensor, or a battery may be disposed on the earphone handle 2. Audio data output by a user may be input to the earphone by using the microphone, and transmitted to an external electronic device connected to the earphone by using the earphone. The control sensor is disposed, and a control action such as a gesture can be performed on the earphone handle 2, to control a working status of the earphone. In addition, a charging contact may be disposed on the earphone handle 2, and the charging contact may be electrically connected to the battery, to implement charging of the battery through the charging contact.

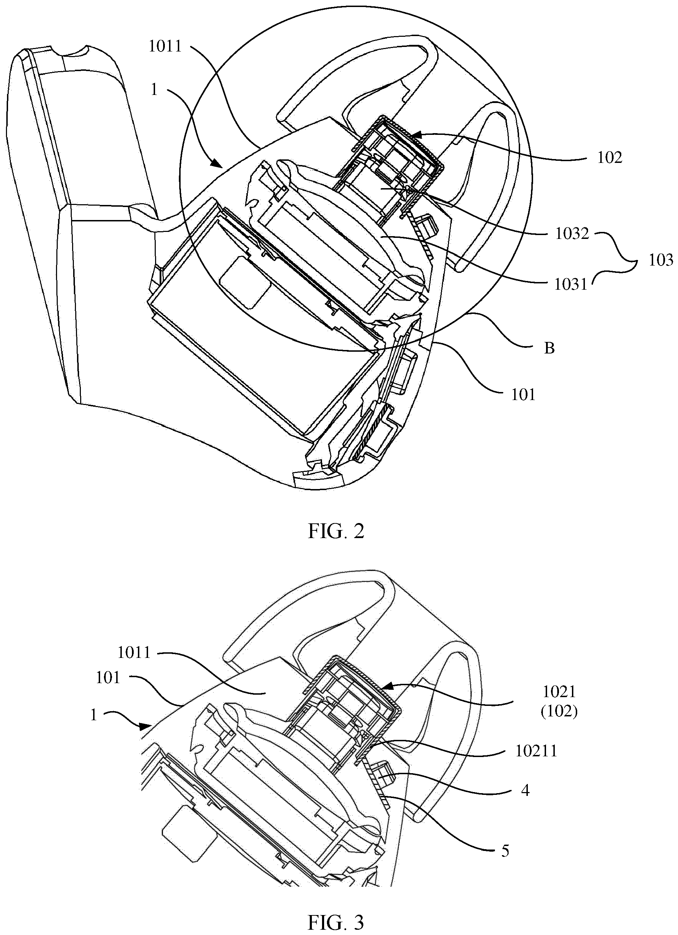

FIG. 2 is a cross sectional view at A-A of the earphone in FIG. 1. It may be understood that, in FIG. 2, to facilitate display of the structure of the earphone, the earphone is rotated by a specific angle based on the earphone in FIG. 1. In addition, FIG. 2 shows a specific structure of the earbud 1. The earbud 1 may include an earbud housing 101 and a sound outlet hole 102 disposed on the earbud housing 101. First accommodating space 1011 may be formed by enclosing the earbud housing 101, and another component of the earbud 1 may be accommodated in the first accommodating space 1011. The sound outlet hole 102 may connect the first accommodating space 1011 and external space of the earphone.

It can be learned from the description of the foregoing embodiment that a temperature of a periosteum of the human ear is closest to a body temperature of the human body. Usually, an ear temperature of the human body is obtained by measuring the temperature of the periosteum. In addition, sound output by the earphone may be listened to by the human ear through vibration of the periosteum. Therefore, when the earphone provided in this application is worn on the human ear, the sound outlet hole 102 may be disposed opposite to the periosteum.

Still refer to FIG. 2. The speaker 103 may be accommodated in the first accommodating space 1011. In addition, in this application, the speaker 103 may include a first sound emitting unit 1031 and a second sound emitting unit 1032. The first sound emitting unit 1031 may be disposed as a moving coil, and the moving coil includes a first diaphragm and a coil disposed on the first diaphragm. The second sound emitting unit 1032 may be disposed as a moving iron, and the moving iron may include a magnetic circuit component and a second diaphragm.

In this application, the moving coil and the moving iron each may be responsible for emitting sound in a different frequency band. It can be learned from FIG. 2 that the second sound emitting unit 1032 of the speaker 103 may be disposed between the first sound emitting unit 1031 and the sound outlet hole 102. When the speaker 103 works, the coil of the moving coil may drive the first diaphragm to vibrate under driving of a signal current. When the first diaphragm vibrates, the first diaphragm may push air disturbance in space between the first diaphragm and the sound outlet hole 102, and produce resonance with air in the space to emit sound. Alternatively, the magnetic circuit component of the moving iron drives the second diaphragm to vibrate. When the second diaphragm vibrates, the second diaphragm may push air disturbance in space between the second diaphragm and the sound outlet hole 102, and produce resonance with air in the space to emit sound. The sound may be transmitted from the inside of the earbud 1 to the outside of the earbud 1 through a hole channel of the sound outlet hole 102, so that the sound is spread in the ear canal of the human ear. Finally, the sound is emitted by the earphone.

Audio effect of the earphone is an important factor for evaluating quality of the earphone. It may be learned from the foregoing description of a sound emitting principle of the earphone that a size of the hole channel of the sound outlet hole 102 affects sound outlet effect of the earphone to some extent. In addition, usually, if an overall size of the earphone is small, the size of the earbud 1 is also small. In addition, because a sound emitting device like the speaker 103 is disposed in the first accommodating space 1011 formed by the earbud housing 101, space for disposing the sound outlet hole 102 in the earbud 1 is small, and the size of the hole channel of the sound outlet hole 102 is limited. Therefore, to make the earphone have better audio effect, another structure is not suitable to be disposed in the hole channel of the sound outlet hole 102.

FIG. 3 is an enlarged view of a partial structure at B in FIG. 2. A first temperature sensor 4 may be disposed on the earphone provided in this application, and the first temperature sensor 4 is disposed in the first accommodating space 1011 of the earbud 1. In addition, the first temperature sensor 4 is disposed on an outer side of the hole channel of the sound outlet hole 102.

In this application, the earbud 1 may be provided with a sound outlet net 1021, and the sound outlet net 1021 may be disposed on an end face of an end that is of the sound outlet hole 102 and that is connected to the external space of the earphone. In this way, the sound outlet net 1021 can also provide a dust-proof function for the sound outlet hole 102 without affecting audio effect of the earphone. In addition, the sound outlet net 1021 may further extend into the first accommodating space 1011 through the sound outlet hole 102.

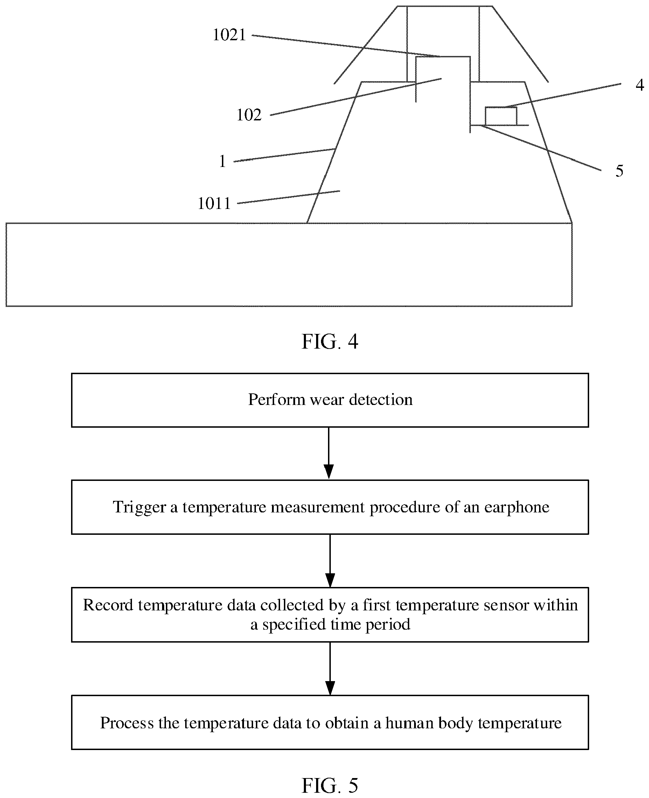

For ease of understanding a specific disposition position of the first temperature sensor 4 of the earphone provided in this application, FIG. 4 is a block diagram of a structure of the earphone according to a possible embodiment of this application. Because the sound outlet hole 102 is disposed towards the periosteum, the temperature of the periosteum can be transferred to the sound outlet hole 102 through air. In a possible embodiment of this application, at least a part of the sound outlet net 1021 may be provided with a material with a high thermally conductive coefficient. For example, the material may be a metal material or a non-metal material with a high thermally conductive coefficient. The metal material with a high thermally conductive coefficient may be but is not limited to copper, stainless steel, nickel, titanium, gold, silver, or the like. In this way, the first temperature sensor 4 may be in thermally conductive contact with the sound outlet hole 102 through the sound outlet net 1021. After the temperature of the periosteum is transferred to the sound outlet hole 102, the temperature may be transferred to the first temperature sensor 4 inside the earphone through the sound outlet net 1021.

It should be noted that, in this application, that at least a part of the sound outlet net 1021 is provided with a material with a high thermally conductive coefficient may be understood as follows: The sound outlet net 1021 as a whole is made of a material with a high thermally conductive coefficient. In this way, each part of the sound outlet net 1021 may be used as a thermally conductive path. Alternatively, a part of the sound outlet net 1021 is made of a material with a high thermally conductive coefficient, and the part formed by the material with a high thermally conductive coefficient may be used as a thermally conductive path of the sound outlet net 1021. Alternatively, a thermally conductive coefficient of the sound outlet net 1021 is not high, but a thermally conductive path is formed by pasting or coating a material with a high thermally conductive coefficient on a surface of the sound outlet net 1021, provided that the temperature of the periosteum can be transferred to the inside of the earphone through the thermally conductive path of the sound outlet net 1021.

Still refer to FIG. 4. The first temperature sensor 4 may be in thermally conductive contact with the thermally conductive path of the sound outlet net 1021, so that the temperature of the periosteum can be transferred to the first temperature sensor 4 through the thermally conductive path of the sound outlet net 1021. During specific implementation, the first temperature sensor 4 may be disposed on a circuit board 5, and the first temperature sensor 4 is electrically connected to the circuit board 5. In this application, a specific type of the circuit board 5 is not limited, and the circuit board 5 may be but is not limited to a printed circuit board (printed circuit board, PCB) or a flexible printed circuit (flexible printed circuit, FPC).

Usually, a large quantity of metal wires may be disposed on the circuit board 5, and the metal wires may be usually formed by metal with a high thermally conductive coefficient, such as copper. In this way, the circuit board 5 can be disposed on the outer side of the hole channel of the sound outlet hole 102, and the circuit board 5 is in thermally conductive contact with an outer side wall of the sound outlet hole 102. In addition, the circuit board 5 may be fastened to an outer side of the sound outlet net 1021 that is enclosed to form the sound outlet hole 102. The circuit board 5 and the sound outlet net 1021 may be fastened through welding, bonding, or the like, to improve reliability of connection between the circuit board 5 and the sound outlet net 1021.

In addition, in this application, the circuit board 5 may be in thermally conductive contact with the thermally conductive path of the sound outlet net 1021, so that the temperature of the periosteum can be transferred to the circuit board 5 through the thermally conductive path of the sound outlet net 1021, and loss of the temperature transferred between the sound outlet net 1021 and the circuit board 5 can be reduced. In addition, because the first temperature sensor 4 is disposed on the circuit board 5, the temperature of the periosteum is transferred to the first temperature sensor 4 after being transferred to the circuit board 5 through the sound outlet net 1021. Therefore, the temperature of the periosteum of the human ear can be obtained by using the first temperature sensor 4, and the temperature may be considered as a human body temperature.

It may be understood that, in this application, to facilitate fastening and connection between the sound outlet net 1021 and the circuit board 5, still refer to FIG. 4. A part of the sound outlet net 1021 that is used to dispose the thermally conductive path may be extended into the inside of the first accommodating space 1011 of the earbud 1, and a specific extension length of the part of the sound outlet net 1021 may be specified according to a disposition position of the circuit board 5 and an internal structure layout of the first accommodating space 1011. In this way, a connection area between the sound outlet net 1021 and the circuit board 5 is increased, and a contact area between the thermally conductive path and the circuit board 5 can also be increased. This improves efficiency of transferring the temperature between the sound outlet net 1021 and the circuit board 5.

In some other possible embodiments of this application, the first temperature sensor 4 may alternatively be directly fastened to the sound outlet net 1021, and the first temperature sensor 4 is in contact with the thermally conductive path of the sound outlet net 1021, so that the temperature of the periosteum can be directly transferred to the first temperature sensor 4 after being transferred to the sound outlet net 1021. This shortens a path for transferring the temperature between the sound outlet net 1021 and the first temperature sensor 4, reduces temperature loss, improves precision of measuring the temperature of the periosteum by the first temperature sensor 4, and improves accuracy of measuring the body temperature by the earphone.

After the structure of the earphone provided in the foregoing embodiments of this application is understood, the following describes a process of performing body temperature measurement by the earphone. FIG. 5 is a flowchart of a body temperature measurement method for the earphone according to an embodiment of this application.

Step 1: Perform wear detection. In this step, a wearing manner of the earphone may be detected by using a wear sensor (for example, an optical proximity sensor or a capacitive sensor) disposed in the earphone. The wearing manner of the earphone includes single-ear wearing or dual-ear wearing.

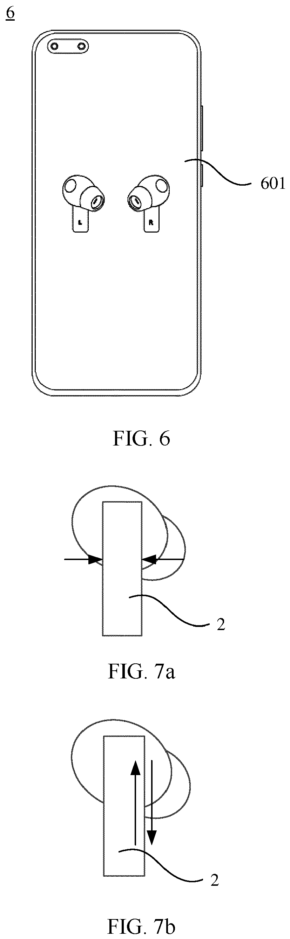

Step 2: Trigger a temperature measurement procedure of the earphone, so that the first temperature sensor 4 starts to collect temperature data. In this application, a manner of triggering the temperature measurement procedure of the earphone is not limited. For example, in a possible embodiment of this application, the earphone may be wiredly or wirelessly connected to the external electronic device 6 shown in FIG. 6, so that signal interaction can be performed between the earphone and the external electronic device 6. The external electronic device 6 may be but is not limited to an electronic device, like a mobile phone, a tablet computer, or a notebook computer, having a display interface 601. In this way, the user can input, on the external electronic device 6, an instruction for starting the temperature measurement procedure of the earphone. A manner of inputting the instruction on the external electronic device 6 may be but is not limited to inputting the instruction on the display interface 601 of the external electronic device 6. After receiving the instruction, a controller in the earphone triggers the temperature measurement procedure of the earphone. In this application, a type of the controller in the earphone is not limited, and the controller may be, for example, a microcontroller unit (microcontroller unit, MCU).

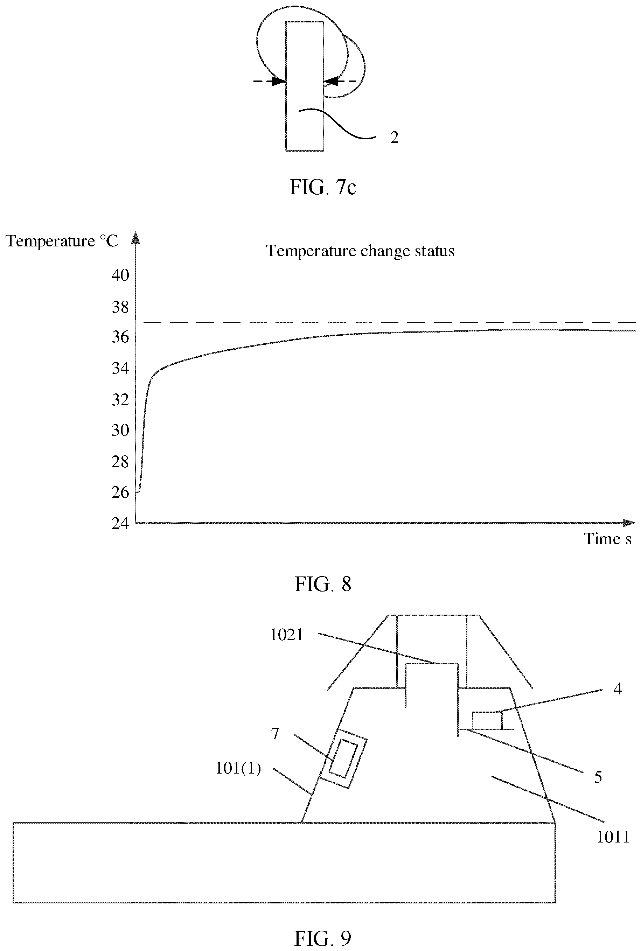

In some other possible embodiments of this application, the controller in the earphone may trigger the temperature measurement procedure when detecting that the earphone is worn on the human ear; or the controller controls the temperature measurement procedure to start after detecting that a single earphone or dual earphones are stably worn on the human ears. Alternatively, refer to FIG. 7a to FIG. 7c. An operation sensor, like a pressure sensor or a touch sensor, is disposed in the earphone, to trigger the temperature measurement procedure by an operation gesture, like long press shown in FIG. 7a (a solid line with an arrow in FIG. 7a represents a pressing direction), slide shown in FIG. 7b (an arrow in FIG. 7b represents a slide direction), or pinch shown in FIG. 7c (a dashed line with an arrow in FIG. 7c represents a touch direction).

Step 3: Record the temperature data collected by the first temperature sensor 4 within a specified time period. During specific implementation, the controller may record, in real time, the temperature collected by the first temperature sensor 4, and draw a temperature data curve. Refer to FIG. 8. In FIG. 8, a solid line is the temperature data curve drawn based on the temperature data collected by the first temperature sensor 4 within the specified time period.

Step 4: Process the temperature data to obtain a human body temperature. During specific implementation, the controller may perform fitting on the temperature data curve obtained in step 3, to obtain a final convergence value of the temperature data curve. In addition, it is considered that body temperatures of users of different ages and genders in different time periods and in different application scenarios have a specific deviation, to improve precision of human body temperature measurement, big data statistics is performed on factors that may affect accuracy of temperature measurement, such as ages, time periods, or application scenarios, to obtain a corresponding bias temperature. In this way, the corresponding bias temperature may be added on the basis of the convergence value, and final temperature data can be obtained. In this application, the bias temperature value may usually range from -5°C to +5°C. For example, the bias temperature value may range from -3°C to +3°C, or from -2.5°C to +2.5°C.

FIG. 8 is used as an example, a final convergence temperature of the solid line in FIG. 8 is 36.5°C, and the temperature is a stable temperature measured by the sensor. A real body temperature of the human body is 37.0°C, as shown by a dashed line in FIG. 8. Therefore, a bias temperature needs to be added. In this embodiment, the bias temperature is 0.5°C. In this way, the real body temperature of the user can be obtained.

In addition, to enable the user to obtain the human body temperature measured by using the earphone, in a possible embodiment of this application, the body temperature measurement process of the earphone provided in this application may further include the following step.

Step 5: Output a body temperature measurement result obtained after processing. Because the speaker 103 is disposed in the earphone, the body temperature measurement result may be broadcast by using the earphone through voice. In addition, it may be learned from the foregoing description of step 1 that the earphone may be connected to the external electronic device 6, like the mobile phone, having the display interface 601. Based on this, the body temperature measurement result may be displayed on the display interface 601 of the external electronic device 6, so that the user can view the result.

It may be understood that, in some embodiments of this application, a memory may be disposed in the electronic device connected to the earphone, to store a body temperature measured by using the earphone in each time. In this way, the user can view the body temperatures at any time, and can learn a health status of the user by comparing body temperature measurement results in a plurality of times.

In addition, the external electronic device 6 may further perform health prompt on a body temperature anomaly measurement result. For example, the external electronic device 6 compares a body temperature measurement result with a predetermined threshold of a normal body temperature. When the body temperature measurement result exceeds the predetermined threshold of the normal body temperature, the external electronic device 6 may mark the body temperature measurement result in a different color or shape, or prompt the user by using a different icon or background, or perform voice health prompt. In addition, when the body temperature measurement result exceeds the predetermined threshold of the normal body temperature, the display interface 601 of the external electronic device 6 may display or prompt a location of a nearest hospital or clinic through voice to the user.

It should be noted that, in this application, before body temperature measurement, the external terminal device 6 may further prompt the user to input parameters that affect a human body temperature attribute, such as an age and a gender, to correct a measured human body temperature and a predetermined threshold of a normal body temperature corresponding to the age and the gender. This obtains a more accurate human body temperature measurement value.

According to the earphone provided in this application, the first temperature sensor 4 is disposed on an outer side surface of the sound outlet hole 102. This can avoid impact on sound outlet effect of the sound outlet hole 102. In addition, a thermally conductive path with a good thermally conductive coefficient is disposed on the sound outlet hole 102, and the first temperature sensor 4 is directly or indirectly connected to the thermally conductive path. Therefore, the temperature of the periosteum of the human ear can be transferred to the sound outlet hole 102 through air, and transferred to the first temperature sensor 4 through the thermally conductive path of the sound outlet hole 102, so that the first temperature sensor 4 can more accurately measure the temperature of the periosteum of the human ear, where the temperature may be considered as a human body temperature, and therefore a more accurate measurement result of the human body temperature is obtained.

Usually, different users have personalized differences in wearing tightness of the earphone. Therefore, to reduce impact caused by the wearing tightness on the body temperature measurement result of the earphone, in a possible embodiment of this application, a second temperature sensor 7 may be disposed on the earphone. FIG. 9 is a block diagram of a structure of the earphone according to another embodiment of this application. In this embodiment, the second temperature sensor 7 is disposed in the first accommodating space 1011 of the earbud 1.

When the earphone is worn on the human ear, a part of the earbud 1 is in contact with skin of a part of the human ear (for example, a concha cavity). Therefore, in this application, the second temperature sensor 7 may be fastened to an inner side wall corresponding to a position at which the earbud housing 101 of the earbud 1 is in contact with human ear skin, so that a human ear skin temperature is transferred to the second temperature sensor 7 by using the earbud housing 101. In addition, to reduce loss of the human ear skin temperature in a transfer process, a part of the earbud housing 101 may be provided with a first thermally conductive portion made of a metal material with a high thermally conductive coefficient. In this way, the first thermally conductive portion can be in contact with the human ear skin, the second temperature sensor 7 is fastened to the first thermally conductive portion through welding or bonding with a thermally conductive adhesive, and the second temperature sensor 7 can be in thermally conductive contact with the first thermally conductive portion. Therefore, the human ear skin temperature can be transferred to the second temperature sensor 7 through the first thermally conductive portion.

It may be understood that the thermally conductive portion may be a structure that is separately disposed on the earbud housing 101 to implement thermal conduction, or may be an original structure that is in the earbud 1 and that is configured to implement a function (like shielding) of the earbud 1. This is not specifically limited in this application.

The temperature of the human ear skin that can be in contact with the earbud 1 is slightly different from that of the periosteum. In addition, the wearing tightness of the earphone on the human ear affects a contact degree between the earphone and the human ear skin, and stability of transferring the human ear skin temperature to the earphone is affected. Therefore, the wearing tightness of the earphone also affects accuracy of measuring the human ear skin temperature by the second temperature sensor 7. It can be learned that accuracy of measuring the human ear skin temperature by the second temperature sensor 7 is affected by the wearing tightness.

Therefore, a calculation method for obtaining the real body temperature of the human body based on the temperature measured by the first temperature sensor 4 and the temperature measured by the second temperature sensor 7 is described below.

It can be learned from the description of the foregoing embodiment that the first temperature sensor 4 is disposed on the outer side of the sound outlet hole 102, and is in contact with the thermally conductive path of the sound outlet hole 102. The measured temperature TA of the first temperature sensor 4 may be expressed as:

$$TA = fa1(T) + fa2(X) \text{ formula } ①$$

In addition, the second temperature sensor 7 is disposed at a part that is of the earbud housing 101 and that is in contact with the human ear skin, and the measured temperature TB of the second temperature sensor 7 may be expressed as:

$$TB = fb1(T) + fb2(X) \text{ formula } ②$$

In this way, the body temperature T of the human body can be obtained by weighting the measured temperature TA of the first temperature sensor 4 and the measured temperature TB of the second temperature sensor 7, that is:

$$T = fa(TA) + fb(TB) \text{ formula } ③$$

In the foregoing formula ①, formula ②, and formula ③, T is the real body temperature of the human body, and X is impact of the wearing tightness on the human body temperature measurement result. fa1, fa2, fb1, fb2, fa and fb are functions, and are components used to map the measured temperature TA of the first temperature sensor 4 and the measured temperature TB of the second temperature sensor 7 in the current application scenario to the user body temperature T.

In this application, these functions fa1, fa2, fb1, fb2, fa and fb may have an expression, or may have no expression. This is not limited in this application, provided that the real body temperature T of the human body can be obtained based on the measured temperature TA of the first temperature sensor 4 and the measured temperature TB of the second temperature sensor 7. In a possible embodiment of this application, when these functions have an expression, the expression may be very simple. For example, the expression may be T = 0.9TA + 0.1TB. In this case, fa and fb are simplest weight coefficients, that is, fa + fb = 1, and fa > fb. In addition, the expression may alternatively be complex, for example, $T = TA^2/\ln(TB)$.

In addition, when these functions have no expression, the functions may be only a mapping relationship table. For example, Table 1 shows a mapping relationship between the measured temperature TA of the first temperature sensor 4 and the measured temperature TB of the second temperature sensor 7. In this way, after the measured temperature TA of the first temperature sensor 4 and the measured temperature TB of the second temperature sensor 7 are obtained, the corresponding real body temperature T of the human body can be obtained by querying the prespecified mapping relationship between the measured temperature TA of the first temperature sensor 4 and the measured temperature TB of the second temperature sensor 7 in Table 1. It may be understood that the mapping relationship table may be prestored in the earphone or in a memory of the external electronic device 6 connected to the earphone, to quickly obtain the corresponding real body temperature T of the human body after the measured temperature TA of the first temperature sensor 4 and the measured temperature TB of the second temperature sensor 7 are obtained.

Table 1

| | | **TA** | | | | | | | | | | | | |
|---|---|------|------|------|------|------|------|------|------|------|------|------|------|------|------|
| | | 35.5 | 36.0 | 36.5 | 37.0 | 37.5 | 38.0 | 38.5 | 39.0 | 39.5 | 40.0 | 40.5 | 41.0 | 41.5 | 42.0 |

(continued)

| TB | | TA | | | | | | | | | | | | | |
|---|---|---|---|---|---|---|---|---|---|---|---|---|---|---|---|
| | **35.5** | 35.5 | 36.0 | 36.4 | 36.9 | 37.3 | 37.8 | 38.2 | 38.7 | 39.1 | 39.6 | 40.0 | 40.5 | 40.9 | 41.4 |
| | **36.0** | 35.6 | 36.0 | 36.5 | 36.9 | 37.4 | 37.8 | 38.3 | 38.7 | 39.2 | 39.6 | 40.1 | 40.5 | 41.0 | 41.4 |
| | **36.5** | 35.6 | 36.1 | 36.5 | 37.0 | 37.4 | 37.9 | 38.3 | 38.8 | 39.2 | 39.7 | 40.1 | 40.6 | 41.0 | 41.5 |
| | **37.0** | 35.7 | 36.1 | 36.6 | 37.0 | 37.5 | 37.9 | 38.4 | 38.8 | 39.3 | 39.7 | 40.2 | 40.6 | 41.1 | 41.5 |
| | **37.5** | 35.7 | 36.2 | 36.6 | 37.1 | 37.5 | 38.0 | 38.4 | 38.9 | 39.3 | 39.8 | 40.2 | 40.7 | 41.1 | 41.6 |
| | **38.0** | 35.8 | 36.2 | 36.7 | 37.1 | 37.6 | 38.0 | 38.5 | 38.9 | 39.4 | 39.8 | 40.3 | 40.7 | 41.2 | 41.6 |
| | **38.5** | 35.8 | 36.3 | 36.7 | 37.2 | 37.6 | 38.1 | 38.5 | 39.0 | 39.4 | 39.9 | 40.3 | 40.8 | 41.2 | 41.7 |
| TB | **39.0** | 35.9 | 36.3 | 36.8 | 37.2 | 37.7 | 38.1 | 38.6 | 39.0 | 39.5 | 39.9 | 40.4 | 40.8 | 41.3 | 41.7 |
| | **39.5** | 35.9 | 36.4 | 36.8 | 37.3 | 37.7 | 38.2 | 38.6 | 39.1 | 39.5 | 40.0 | 40.4 | 40.9 | 41.3 | 41.8 |
| | **40.0** | 36.0 | 36.4 | 36.9 | 37.3 | 37.8 | 38.2 | 38.7 | 39.1 | 39.6 | 40.0 | 40.5 | 40.9 | 41.4 | 41.8 |
| | **40.5** | 36.0 | 36.5 | 36.9 | 37.4 | 37.8 | 38.3 | 38.7 | 39.2 | 39.6 | 40.1 | 40.5 | 41.0 | 41.4 | 41.9 |
| | **41.0** | 36.1 | 36.5 | 37.0 | 37.4 | 37.9 | 38.3 | 38.8 | 39.2 | 39.7 | 40.1 | 40.6 | 41.0 | 41.5 | 41.9 |
| | **41.5** | 36.1 | 36.6 | 37.0 | 37.5 | 37.9 | 38.4 | 38.8 | 39.3 | 39.7 | 40.2 | 40.6 | 41.1 | 41.5 | 42.0 |
| | **42.0** | 36.2 | 36.6 | 37.1 | 37.5 | 38.0 | 38.4 | 38.9 | 39.3 | 39.8 | 40.2 | 40.7 | 41.1 | 41.6 | 42.0 |

In this embodiment of this application, both the first temperature sensor 4 and the second temperature sensor 7 are disposed in the earphone, to measure the temperature of the periosteum by using the first temperature sensor 4, and measure the human ear skin temperature by using the second temperature sensor 7. In addition, according to different degrees of impact of the wearing tightness of the earphone on the first temperature sensor 4 and the second temperature sensor 7, a body temperature value of the human body is obtained through weighting based on a function relationship between the temperature measured by the first temperature sensor 4 and the wearing tightness of the earphone and the body temperature of the human body, and a function relationship between the temperature measured by the second temperature sensor 7 and the wearing tightness of the earphone and the body temperature of the human body, to eliminate impact of the wearing tightness on the body temperature of the human body. In this way, a requirement of earphone temperature measurement on the wearing tightness of the earphone is eliminated, to implement accurate temperature measurement in a comfortable and natural wearing status of the user.

In addition, in different application scenarios (such as indoor office, outdoor exercise, normal temperature, low temperature, or high temperature), expressions or mapping relationship tables of a same function are different. Therefore, for different application scenarios, actual body temperatures of a large quantity of users, the temperature measured by the first temperature sensor 4, and the temperature measured by the second temperature sensor 7 may be collected, and the collected temperatures are substituted into the foregoing formula ①, formula (2), and formula (3), to obtain expressions or mapping relationship tables of the six functions fa1, fa2, fb1, fb2, fa and fb in different application scenarios. Table 2 shows the expressions or the mapping relationship tables of the six functions fa1, fa2, fb1, fb2, fa and fb in different application scenarios provided in a possible embodiment of this application.

**Table 2**

| Indoor office | Normal temperature | fa1 = ... | fa2 = ... | fb1 = ... | fb2 = ... | fa = ... | fb = ... |
|---|---|---|---|---|---|---|---|
| | Low temperature | fa1 = ... | fa2 = ... | fb1 = ... | fb2 = ... | fa = ... | fb = ... |
| | High temperature | fa1 = ... | fa2 = ... | fb1 = ... | fb2 = ... | fa = ... | fb = ... |
| Outdoor exercise | Normal temperature | fa1 = ... | fa2 = ... | fb1 = ... | fb2 = ... | fa = ... | fb = ... |
| | Low temperature | fa1 = ... | fa2 = ... | fb1 = ... | fb2 = ... | fa = ... | fb = ... |
| | High temperature | fa1 = ... | fa2 = ... | fb1 = ... | fb2 = ... | fa = ... | fb = ... |
| ... | ... | ... | | | | | |

It may be understood that, in Table 2, corresponding to different application scenarios, expressions or mapping relationship tables of a same function are different. For example, fa1 = 0.8 at a normal temperature, and fa1 = 0.9 at a low temperature.

In this way, during actual application of the earphone provided in this application, a current application scenario of the user may be inferred by monitoring a behavior habit of the user or by disposing another sensor, and expressions or mapping relationship tables of corresponding functions can be obtained by querying Table 2. Therefore, the real body temperature T of the user can be obtained by substituting the temperature TA measured by the first temperature sensor 4 and the temperature TB measured by the second temperature sensor 7.

In addition, after the real body temperature T of the user is obtained, the temperature TA measured by the first temperature sensor 4 and the real body temperature T of the user may be substituted into the foregoing formula ①, or the temperature TB measured by the second temperature sensor 7 and the real body temperature T of the user may be substituted into the foregoing formula ②, to obtain the impact X of the wearing tightness on the body temperature measurement result of the human body. Therefore, the wearing behavior habit of the user can be evaluated based on the impact X of the wearing tightness on the body temperature measurement result of the human body, and guidance and correction can be performed on the wearing behavior habit. It should be noted that the guidance or correction process may be implemented by using, but is not limited to, voice broadcast of the earphone, or may be displayed by using an interface of the external electronic device 6 connected to the earphone.

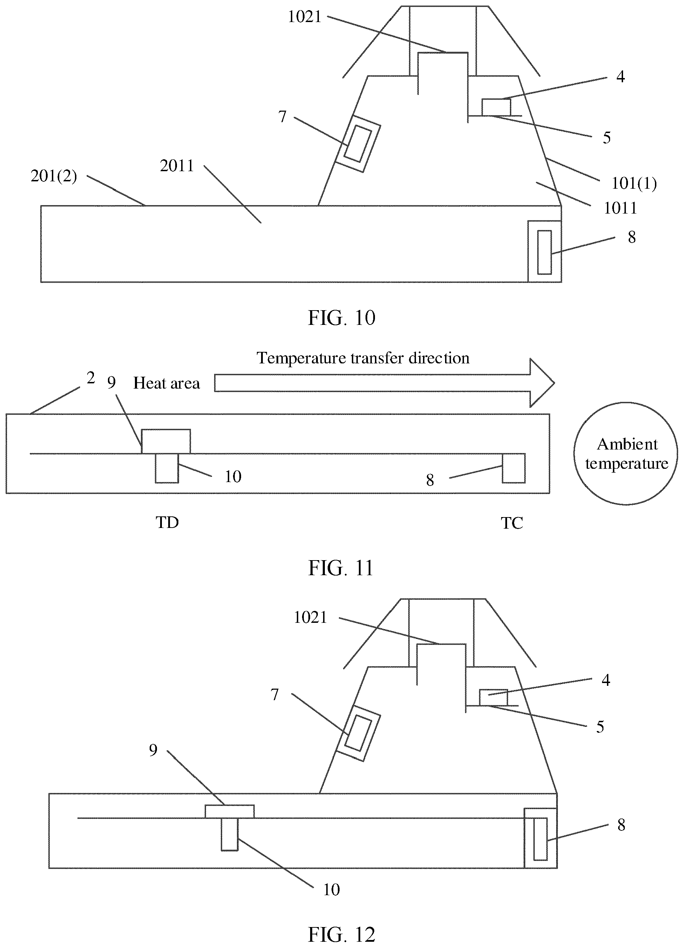

As it is known, an external ambient temperature also affects the temperature measurement process of the earphone. Therefore, in a possible embodiment of this application, based on the first temperature sensor 4 and the second temperature sensor 7, a third temperature sensor 8 may be disposed on the earphone. The third temperature sensor 8 may be configured to measure the ambient temperature, to reduce an error in body temperature measurement at different ambient temperatures, and improve accuracy of body temperature measurement of the human body performed by the earphone. During specific implementation, FIG. 10 is a block diagram of a structure of the earphone according to another possible embodiment of this application. In this embodiment, the third temperature sensor 8 may be disposed on the earphone handle 2. The earphone handle 2 may include an earphone handle housing 201, and second accommodating space 2011 is formed by enclosing the earphone handle housing 201. In this case, the third temperature sensor 8 may be disposed in the second accommodating space 2011, and is fastened to an inner side wall corresponding to a position at which the earphone handle housing 201 is in contact with the external space of the earphone.

In addition, to reduce loss of the ambient temperature in the transfer process, a part of the earphone handle housing 201 may be provided with a second thermally conductive portion made of a metal material with a high thermally conductive coefficient. In this way, the third temperature sensor 8 can be fastened to the second thermally conductive portion through welding or bonding with a thermally conductive adhesive, and the third temperature sensor 8 can be in thermally conductive contact with the second thermally conductive portion. Therefore, the ambient temperature can be transferred to the third temperature sensor 8 through the second thermally conductive portion.

It may be understood that the thermally conductive portion of the earphone handle 2 may be a structure that is separately disposed on the earphone handle housing 201 to implement thermal conduction, or may be an original structure that is in the earphone handle 2 and that is configured to implement a function (like shielding) of the earphone handle 2. This is not specifically limited in this application.

In another possible embodiment of this application, the third temperature sensor 8 may be disposed in the first accommodating space 1011 of the earbud 1, and the third temperature sensor 8 is fastened to the inner side wall of the earbud housing 101, provided that the third temperature sensor 8 can measure the ambient temperature.

In this embodiment of this application, a calculation method for obtaining the real body temperature of the human body based on the temperature measured by the first temperature sensor 4, the temperature measured by the second temperature sensor 7, and the temperature measured by the third temperature sensor 8 is described below.

It can be learned from the description of the foregoing embodiment that the first temperature sensor 4 is disposed on the outer side of the sound outlet hole 102, and is in contact with the thermally conductive path of the sound outlet hole 102. The temperature TA measured by the first temperature sensor 4 is affected by the ambient temperature and the wearing tightness, and the measured temperature TA of the first temperature sensor 4 may be expressed as:

$$TA = fa1(T) + fa2(H) + fa3(X) \text{ formula } ①$$

In addition, the second temperature sensor 7 is disposed at a part that is of the housing and that is in contact with the human ear skin, and the temperature TB measured by the second temperature sensor 7 is mainly affected by the ambient temperature and the wearing tightness. Therefore, the measured temperature TB of the second temperature sensor 7 may be expressed as:

$$TB = fb1(T) + fb2(H) + fb3(X) \text{ formula } ②$$

The third temperature sensor 8 is configured to measure the ambient temperature, and the third temperature sensor 8 is mainly affected by the ambient temperature. Therefore, the measured temperature TC of the third temperature sensor 8 may be expressed as:

$$TC = fc1(H) \text{ formula } ③$$

In this way, the real body temperature of the human body can be obtained by weighting the measured temperature TA of the first temperature sensor 4, the measured temperature TB of the second temperature sensor 7, and the measured temperature TC of the second temperature sensor 7, that is:

$$T = fa(TA) + fb(TB) + fc(TC) \text{ formula } ④$$

In the foregoing formula ①, formula ②, formula ③, and formula ④, T is a real body temperature of the human body, X is impact of wearing tightness on a body temperature measurement result of the human body, and H is impact of an ambient temperature on the body temperature measurement result. In addition, fa1, fa2, fa3, fb1, fb2, fb3, fc1, fa, fb and fc are functions, and are components used to map the temperature TA measured by the first temperature sensor 4, the temperature TB measured by the second temperature sensor 7, and the temperature TC measured by the third temperature sensor 8 in the current application scenario to the user body temperature T.

In this application, these functions fa1, fa2, fa3, fb1, fb2, fb3, fc1, fa, fb and fc may have an expression, or may have no expression. This is not limited in this application, provided that the real body temperature T of the human body can be obtained based on the measured temperature TA of the first temperature sensor 4, the measured temperature TB of the second temperature sensor 7, and the measured temperature TC of the third temperature sensor 8. In a possible embodiment of this application, when these functions have an expression, the expression may be very simple. For example, the expression may be T = 0.8TA + 0.1TB + 0.1TC. In this case, fa, fb, and fc are simplest weight coefficients, fa + fb + fc = 1, and fa > fb. In addition, the expression may alternatively be complex, for example, T = TA2/ln(TB) + lg(TC).

In addition, these functions may have no expression. When these functions have no expression, the functions may be only a mapping relationship table. For example, refer to Table 3 and Table 4. Table 3 shows a mapping relationship between the measured temperature TA of the first temperature sensor 4 and the measured temperature TB of the second temperature sensor 7. The temperature TAB can be obtained based on the mapping relationship in Table 3. In addition, Table 4 shows a mapping relationship between the temperature TAB and the measured temperature TC of the third temperature sensor 8. The real body temperature T of the human body can be obtained based on the mapping relationship in Table 4.

In this way, after the measured temperature TA of the first temperature sensor 4 and the measured temperature TB of the second temperature sensor 7 are obtained, the corresponding temperature TAB can be obtained by querying the specified mapping relationship between the measured temperature TA of the first temperature sensor 4 and the measured temperature TB of the second temperature sensor 7 in Table 3. Then, the corresponding real body temperature T of the human body can be obtained based on the specified mapping relationship between the temperature TAB and the measured temperature TC of the third temperature sensor 8 in Table 4. It may be understood that, in this application, Table 3 and Table 4 may be prestored in the earphone or in a memory of the external electronic device 6 connected to the earphone, to quickly obtain the corresponding real body temperature T of the human body after the measured temperature TA of the first temperature sensor 4, the measured temperature TB of the second temperature sensor 7, and the measured temperature TC of the third temperature sensor 8 are obtained.

**Table 3**

| | | 35.5 | 36.0 | 36.5 | 37.0 | 37.5 | 38.0 | 38.5 | 39.0 | 39.5 | 40.0 | 40.5 | 41.0 | 41.5 | 42.0 |
|---|---|---|---|---|---|---|---|---|---|---|---|---|---|---|---|
| | | | | | | | | TA | | | | | | | |
| | **35.5** | 35.5 | 36.0 | 36.4 | 36.9 | 37.3 | 37.8 | 38.2 | 38.7 | 39.1 | 39.6 | 40.0 | 40.5 | 40.9 | 41.4 |
| | **36.0** | 35.6 | 36.0 | 36.5 | 36.9 | 37.4 | 37.8 | 38.3 | 38.7 | 39.2 | 39.6 | 40.1 | 40.5 | 41.0 | 41.4 |
| | **36.5** | 35.6 | 36.1 | 36.5 | 37.0 | 37.4 | 37.9 | 38.3 | 38.8 | 39.2 | 39.7 | 40.1 | 40.6 | 41.0 | 41.5 |
| | **37.0** | 35.7 | 36.1 | 36.6 | 37.0 | 37.5 | 37.9 | 38.4 | 38.8 | 39.3 | 39.7 | 40.2 | 40.6 | 41.1 | 41.5 |
| | **37.5** | 35.7 | 36.2 | 36.6 | 37.1 | 37.5 | 38.a | 38.4 | 38.9 | 39.3 | 39.8 | 40.2 | 40.7 | 41.1 | 41.6 |
| | **38.0** | 35.8 | 36.2 | 36.7 | 37.1 | 37.6 | 38.0 | 38.5 | 38.9 | 39.4 | 39.8 | 40.3 | 40.7 | 41.2 | 41.6 |
| **TB** | **38.5** | 35.8 | 36.3 | 36.7 | 37.2 | 37.6 | 38.3 | 38.5 | 39.3 | 39.4 | 39.9 | 40.3 | 40.8 | 41.2 | 41.7 |
| | **39.0** | 35.9 | 36.3 | 36.8 | 37.2 | 37.7 | 38.3 | 38.6 | 39.2 | 39.5 | 39.9 | 40.4 | 40.8 | 41.3 | 41.7 |
| | **39.5** | 35.9 | 36.4 | 36.8 | 37.3 | 37.7 | 38.2 | 38.6 | 39.1 | 39.5 | 40.0 | 40.4 | 40.9 | 41.3 | 41.8 |
| | **40.0** | 36.0 | 36.4 | 36.9 | 37.3 | 37.8 | 38.2 | 38.7 | 39.1 | 39.6 | 40.0 | 40.5 | 40.9 | 41.4 | 41.8 |
| | **40.5** | 36.0 | 36.5 | 36.9 | 37.4 | 37.8 | 38.3 | 38.7 | 39.2 | 39.6 | 40.1 | 40.5 | 41.0 | 41.4 | 41.9 |
| | **41.0** | 36.1 | 36.5 | 37.0 | 37.4 | 37.9 | 38.3 | 38.8 | 39.2 | 39.7 | 40.1 | 40.6 | 41.0 | 41.5 | 41.9 |
| | **41.5** | 36.1 | 36.6 | 37.0 | 37.5 | 37.9 | 38.4 | 38.8 | 39.3 | 39.7 | 40.2 | 40.6 | 41.1 | 41.5 | 42.0 |
| | **42.0** | 36.2 | 36.6 | 37.1 | 37.5 | 38.0 | 38.4 | 38.9 | 39.3 | 39.8 | 40.2 | 40.7 | 41.1 | 41.6 | 42.0 |

**Table 4**

| | | 35.5 | 36.0 | 36.5 | 37.0 | 37.5 | 38.0 | 38.5 | 39.0 | 39.5 | 40.0 | 40.5 | 41.0 | 41.5 | 42.0 |
|---|---|---|---|---|---|---|---|---|---|---|---|---|---|---|---|
| | | | | | | | | TAB | | | | | | | |
| | **0.0** | 35.5 | 36.a | 36.4 | 36.9 | 37.3 | 37.8 | 38.2 | 38.7 | 39.1 | 39.6 | 40.0 | 40.5 | 40.9 | 41.4 |
| | **5.0** | 35.6 | 36.a | 36.5 | 36.9 | 37.4 | 37.8 | 38.3 | 38.7 | 39.2 | 39.6 | 40.1 | 40.5 | 41.0 | 41.4 |
| | **10.0** | 35.6 | 36.1 | 36.5 | 37.0 | 37.4 | 37.9 | 38.3 | 38.8 | 39.2 | 39.7 | 40.1 | 40.6 | 41.0 | 41.5 |
| | **15.0** | 35.7 | 36.1 | 36.6 | 37.0 | 37.5 | 37.9 | 38.4 | 38.8 | 39.3 | 39.7 | 40.2 | 40.6 | 41.1 | 41.5 |
| | **20.0** | 35.7 | 36.2 | 36.6 | 37.1 | 37.5 | 38.0 | 38.4 | 38.9 | 39.3 | 39.8 | 40.2 | 40.7 | 41.1 | 41.6 |
| | **25.0** | 35.8 | 36.2 | 36.7 | 37.1 | 37.6 | 38.0 | 38.5 | 38.9 | 39.4 | 39.8 | 40.3 | 40.7 | 41.2 | 41.6 |
| **TC** | **28.0** | 35.8 | 36.3 | 36.7 | 37.2 | 37.6 | 38.1 | 38.5 | 39.0 | 39.4 | 39.9 | 40.3 | 40.8 | 41.2 | 41.7 |
| | **30.0** | 35.9 | 36.3 | 36.8 | 37.2 | 37.7 | 38.1 | 38.6 | 39.0 | 39.5 | 39.9 | 40.4 | 40.8 | 41.3 | 41.7 |
| | **35.0** | 35.9 | 36.4 | 36.8 | 37.3 | 37.7 | 38.2 | 38.6 | 39.1 | 39.5 | 40.0 | 40.4 | 40.9 | 41.3 | 41.8 |
| | **40.0** | 36.0 | 36.4 | 36.9 | 37.3 | 37.8 | 38.2 | 38.7 | 39.1 | 39.6 | 40.0 | 40.5 | 40.9 | 41.4 | 41.8 |
| | **45.0** | 36.0 | 36.5 | 36.9 | 37.4 | 37.8 | 38.3 | 38.7 | 39.2 | 39.6 | 40.1 | 40.5 | 41.0 | 41.4 | 41.9 |
| | **50.0** | 36.1 | 36.5 | 37.0 | 37.4 | 37.9 | 38.3 | 38.8 | 39.2 | 39.7 | 40.1 | 40.6 | 41.0 | 41.5 | 41.9 |
| | **55.0** | 36.1 | 36.6 | 37.0 | 37.5 | 37.9 | 38.4 | 38.8 | 39.3 | 39.7 | 40.2 | 40.6 | 41.1 | 41.5 | 42.0 |
| | **60.0** | 36.2 | 36.6 | 37.1 | 37.5 | 38.0 | 38.4 | 38.9 | 39.3 | 39.8 | 40.2 | 40.7 | 41.1 | 41.6 | 42.0 |

In this embodiment of this application, the first temperature sensor 4, the second temperature sensor 7, and the third temperature sensor 8 are all disposed in the earphone, to measure the temperature of the periosteum by using the first temperature sensor 4, measure the human ear skin temperature by using the second temperature sensor 7, and measure the ambient temperature by using the third temperature sensor 8. In addition, according to different degrees of impact of the wearing tightness of the earphone and the ambient temperature on the first temperature sensor 4 and the second temperature sensor 7, a real body temperature value of the human body is obtained through weighting based on a function relationship between the temperature measured by the first temperature sensor 4, the wearing tightness of the earphone, the ambient temperature, and the body temperature of the human body, a function relationship between the temperature measured by the second temperature sensor 7, the wearing tightness of the earphone, the ambient temperature, and the body temperature of the human body, and the ambient temperature measured by the third temperature sensor 8, to eliminate impact of the wearing tightness and the ambient temperature on body temperature measurement of the human body. In this way, a requirement of earphone temperature measurement on the wearing tightness of the earphone and impact of the ambient temperature on earphone temperature measurement are eliminated, to implement accurate temperature measurement in a comfortable and natural wearing status of the user and at various ambient temperatures.

In different application scenarios (such as indoor office, outdoor exercise, normal temperature, low temperature, or high temperature), expressions or mapping relationship tables of a same function are different. Therefore, for different application scenarios, actual body temperatures of a large quantity of users, the temperature measured by the first temperature sensor 4, the temperature measured by the second temperature sensor 7, and the temperature measured by the third temperature sensor 8 may be collected, and the collected temperatures are substituted into the foregoing formula ①, formula ②, formula ③, and formula ④, to obtain expressions or mapping relationship tables of the nine functions fa1, fa2, fa3, fb1, fb2, fb3, fc1, fa, fb and fc in different scenarios. Table 5 shows the expressions or the mapping relationship tables of the nine functions fa1, fa2, fa3, fb1, fb2, fb3, fc1, fa, fb and fc in different application scenarios provided in a possible embodiment of this application.

**Table 5**

| Indoor office | Normal temperature | fa1 = ... fa2 = ... fa3 = ... fb1 = ... fb2 = ... fb3 = ... fa = ... fb = ... fc= ... |
|---|---|---|
| | Low temperature | fa1 = ... fa2 = ... fa3 = ... fb1 = ... fb2 = ... fb3 = ... fa = ... fb = ... fc = ... |
| | High temperature | fa1 = ... fa2 = ... fa3 = ... fb1 = ... fb2 = ... fb3 = ... fa = ... fb = ... fc = ... |
| Outdoor exercise | Normal temperature | fa1 = ... fa2 = ... fa3 = ... fb1 = ... fb2 = ... fb3 = ... fa = ... fb = ... fc= ... |
| | Low temperature | fa1 = ... fa2 = ... fa3 = ... fb1 = ... fb2 = ... fb3 = ... fa = ... fb = ... fc= ... |
| | High temperature | fa1 = ... fa2 = ... fa3 = ... fb1 = ... fb2 = ... fb3 = ... fa = ... fb = ... fc = ... |
| ... | ... | ... |

It may be understood that, in Table 5, corresponding to different application scenarios, expressions or mapping relationship tables of a same function are different. For example, fa1 = 0.8 at a normal temperature, and fa1 = 0.9 at a low temperature.

In this way, during actual application of the earphone provided in this application, a current application scenario of the user may be inferred by monitoring a behavior habit of the user or by disposing another sensor, and expressions or mapping relationship tables of corresponding functions can be obtained by querying Table 5. Therefore, the real body temperature T of the user can be obtained by substituting the temperature TA measured by the first temperature sensor 4, the temperature TB measured by the second temperature sensor 7, and the temperature TC measured by the third temperature sensor 8.

In addition, after the real body temperature T of the user is obtained, the temperature TA measured by the first temperature sensor 4, the real body temperature T of the user, and the temperature TC measured by the third temperature sensor 8 may be substituted into the foregoing formula ① or formula ②, to obtain the impact X of the wearing tightness on the body temperature measurement result of the human body. Therefore, the wearing behavior habit of the user can be evaluated based on the impact X of the wearing tightness on the body temperature measurement result of the human body, and guidance and correction can be performed on the wearing behavior habit. It should be noted that the guidance or correction process may be implemented by using, but is not limited to, voice broadcast of the earphone, or may be displayed by using the display interface of the external electronic device 6 connected to the earphone.

Usually, a large quantity of heat emitting elements, such as the controller mentioned above, are disposed in the earphone. In a working process, temperatures of these heat emitting elements gradually increase, and it is difficult to diffuse the temperatures to the outside of the earphone in time, and the temperatures may converge inside the earphone. When a temperature TD of the heat emitting element is higher than an ambient temperature H, that is, TD > H, heat is transferred from high to low places according to the second law of thermodynamics. FIG. 11 is a schematic diagram of a heat transfer path according to an embodiment of this application. The third temperature sensor 8 is configured to measure the ambient temperature, and is located on the heat transfer path. Therefore, the temperature measured by the third temperature sensor 8 is definitely affected by both a heat emitting element 9 and the ambient temperature.

To reduce impact of heat generated by the heat emitting element 9 on the temperature measured by the third temperature sensor 8, in a possible embodiment of this application, in addition to the first temperature sensor 4, the second temperature sensor 7, and the third temperature sensor 8, the earphone may be provided with a fourth temperature sensor 10. It may be understood that, in this application, the third temperature sensor 8 and the fourth temperature sensor 10 are spaced from each other. FIG. 12 is a block diagram of a structure of the earphone according to another possible embodiment of this application. In this embodiment, the heat emitting element 9 may be disposed in the first accommodating space 1011 and/or the second accommodating space 2011, the fourth temperature sensor 10 may be disposed in the first accommodating space 1011 and/or the second accommodating space 2011, and the fourth temperature sensor 10 is disposed close to the heat emitting element 9. For example, the fourth temperature sensor 10 and the heat emitting element 9 may be disposed on a same circuit board. Alternatively, the fourth temperature sensor 10 and the heat emitting element 9 may be separately disposed on two sides of a same circuit board, and the fourth temperature sensor 10 is disposed facing away from the heat emitting element 9, so that the fourth temperature sensor 10 can more accurately measure the temperature of the heat emitting element 9.

In this embodiment, the temperature TA measured by the first temperature sensor 4 is mainly affected by the ambient temperature and the wearing tightness, and the measured temperature TA of the first temperature sensor 4 may be expressed as:

$$TA = fa1(T) + fa2(H) + fa3(X) \text{ formula } ①$$

In addition, the second temperature sensor 7 is disposed at a part that is of the housing and that is in contact with the human ear skin, and the temperature TB measured by the second temperature sensor 7 is mainly affected by the ambient temperature and the wearing tightness. Therefore, the measured temperature TB of the second temperature sensor 7 may be expressed as:

$$TB = fb1(T) + fb2(H) + fb3(X) \text{ formula } ②$$

In this embodiment, the third temperature sensor 8 is configured to measure the ambient temperature, and the third temperature sensor 8 is mainly affected by the ambient temperature and the temperature of the heat emitting element 9. Therefore, the measured temperature TC of the third temperature sensor 8 may be expressed as:

$$TC = fc1(H) + fc2(TD) \text{ formula } ③$$

In the foregoing formula ③, TD is the temperature of the heat emitting element 9 measured by the fourth temperature sensor 10. In this embodiment of this application, if only the temperature measured by the third temperature sensor 8 is used to estimate the ambient temperature H, a large error is caused to the real body temperature of the human body calculated according to the formulas because temperatures TDs of the heat emitting element 9 measured by the fourth temperature sensor 10 are different. To reduce impact of the heat emitting element 9 on body temperature measurement, the ambient temperature H may be expressed as:

$$H = fc(TC) + fd(TD) \text{ formula } ④$$

In the foregoing formula ③ and formula ④, fc1, fc2, fc, and fd are functions, and are components used to map the temperature TC measured by the third temperature sensor 8 and the temperature TD measured by the fourth temperature sensor 10 in the current application scenario to the ambient temperature H.

In this application, these functions fc1, fc2, fc, and fd may have an expression, or may have no expression. This is not limited in this application, provided that the ambient temperature H can be obtained based on the measured temperature TC of the third temperature sensor 8 and the measured temperature TD of the fourth temperature sensor 10. In a possible embodiment of this application, when these functions have an expression, the expression may be very simple. For example, $H = 0.9TC + 0.1TD$. In this case, fc and fd are simplest weight coefficients, $fc + fd = 1$, and $fc > fd$. In addition, the expression may alternatively be complex, for example, $H = TC^2/\ln(TD)$.

In addition, these functions may alternatively have no expression. When these functions have no expression, the functions may be only a mapping relationship table. For example, Table 6 shows a mapping relationship between the measured temperature TC of the third temperature sensor 8 and the measured temperature TD of the fourth temperature sensor 10. In this way, after the measured temperature TC of the third temperature sensor 8 and the measured temperature TD of the fourth temperature sensor 10 are obtained, the corresponding ambient temperature H can be obtained by querying the specified mapping relationship between the measured temperature TC of the third temperature sensor 8 and the measured temperature TD of the fourth temperature sensor 10 in Table 6. It may be understood that the mapping relationship table may be prestored in the earphone or in a memory of the external electronic device 6 connected to the earphone, to quickly obtain the corresponding ambient temperature H after the measured temperature TC of the third temperature sensor 8 and the measured temperature TD of the fourth temperature sensor 10 are obtained.

**Table 6**

| | | TC | | | | | | | | | | | | | |
|---|---|---|---|---|---|---|---|---|---|---|---|---|---|---|---|
| | | 0.0 | 5.0 | 10.0 | 15.0 | 20.0 | 25.0 | 28.0 | 30.0 | 35.0 | 40.0 | 45.0 | 50.0 | 55.0 | 60.0 |
| | **0.0** | 0.0 | 4.5 | 9.0 | 13.5 | 18.0 | 22.5 | 25.2 | 27.0 | 31.5 | 36.0 | 40.5 | 45.0 | 49.5 | 54.0 |
| | **5.0** | 0.5 | 5.0 | 9.5 | 14.0 | 18.5 | 23.0 | 25.7 | 27.5 | 32.0 | 36.5 | 41.0 | 45.5 | 50.0 | 54.5 |
| | **10.0** | 1.0 | 5.5 | 10.0 | 14.5 | 19.0 | 23.5 | 26.2 | 28.0 | 32.5 | 37.0 | 41.5 | 46.0 | 50.5 | 55.0 |
| | **15.0** | 1.5 | 6.0 | 10.5 | 15.0 | 19.5 | 24.0 | 26.7 | 28.5 | 33.0 | 37.5 | 42.a | 46.5 | 51.0 | 55.5 |
| | **20.0** | 2.0 | 6.5 | 11.0 | 15.5 | 20.0 | 24.5 | 27.2 | 29.0 | 33.5 | 38.0 | 42.5 | 47.0 | 51.5 | 56.0 |
| | **25.0** | 2.5 | 7.0 | 11.5 | 16.0 | 20.5 | 25.0 | 27.7 | 29.5 | 34.0 | 38.5 | 43.0 | 47.5 | 52.0 | 56.5 |
| **TD** | **28.0** | 2.8 | 7.3 | 11.8 | 16.3 | 20.8 | 25.3 | 28.0 | 29.8 | 34.3 | 38.8 | 43.3 | 47.8 | 52.3 | 56.8 |
| | **30.0** | 3.0 | 7.5 | 12.0 | 16.5 | 21.0 | 25.5 | 28.2 | 30.0 | 34.5 | 39.0 | 43.5 | 48.0. | 52.5 | 57.0 |
| | **35.0** | 3.5 | 8.0 | 12.5 | 17.0 | 21.5 | 26.0 | 28.7 | 30.5 | 35.0 | 39.5 | 44.0 | 48.5 | 53.0 | 57.5 |
| | **40.0** | 4.0 | 8.5 | 13.0 | 17.5 | 22.0 | 26.5 | 29.2 | 31.0 | 35.5 | 40.0 | 44.5 | 49.0 | 53.5 | 58.0 |
| | **45.0** | 4.5 | 9.0 | 13.5 | 18.0 | 22.5 | 27.0 | 29.7 | 31.5 | 36.0 | 40.5 | 45.0 | 49.5 | 54.0 | 58.5 |
| | **50.0** | 5.0 | 9.5 | 14.0 | 18.5 | 23.0 | 27.5 | 30.2 | 32.0 | 36.5 | 41.0 | 45.5 | 50.0 | 54.5 | 59.0 |
| | **55.0** | 5.5 | 10.0 | 14.5 | 19.0 | 23.5 | 28.0 | 30.7 | 32.5 | 37.0 | 41.5 | 46.0 | 50.5 | 55.0 | 59.5 |
| | **60.0** | 6.0 | 10.5 | 15.0 | 19.5 | 24.0 | 28.5 | 31.2 | 33.0 | 37.5 | 42.0 | 46.5 | 51.055.5 | 55.5 | 60.0 |

In different application scenarios (such as listening to music, calling, normal temperature, low temperature, or high temperature), expressions or mapping relationship tables of a same function are different. Therefore, for different application scenarios, a large quantity of temperatures measured by the third temperature sensor 8 and a large quantity of temperatures measured by the fourth temperature sensor 10 may be collected, and the collected temperatures are substituted into the foregoing formula ③ and formula ④, to obtain expressions or mapping relationship tables of the four functions fc1, fc2, fc, and fd in different scenarios. Table 7 shows the expressions or the mapping relationship tables of the four functions fc1, fc2, fc, and fd in different application scenarios provided in a possible embodiment of this application.

**Table 7**

| Listening to music | Normal temperature | fc1 = ... | fc2 = ... | fc = ... | fd = ... |
|---|---|---|---|---|---|
| | Low temperature | fc1 = ... | fc2 = ... | fc = ... | fd = ... |
| | High temperature | fc1 = ... | fc2 = ... | fc = ... | fd = ... |
| Calling | Normal temperature | fc1 = ... | fc2 = ... | fc = ... | fd = ... |
| | Low temperature | fc1 = ... | fc2 = ... | fc = ... | fd = ... |
| | High temperature | fc1 = ... | fc2 = ... | fc = ... | fd = ... |
| ... | ... | ... | | | |

It may be understood that, in Table 7, corresponding to different application scenarios, expressions or mapping relationship tables of a same function are different. For example, fc = 0.8 at a normal temperature, and fc = 0.9 at a low temperature.

In this way, during actual application of the earphone provided in this application, a corresponding function expression can be obtained by querying a table according to a current application scenario, and the corresponding function expression may be substituted into the foregoing formula ③ and formula ④, to obtain the current ambient temperature H. In addition, after the ambient temperature H is obtained, the ambient temperature H may be substituted into a functional relational expression between the temperature TA measured by the first temperature sensor 4 and the ambient temperature H and the wearing tightness X, and a functional relational expression between the temperature TB measured by the second temperature sensor 7 and the ambient temperature H and the wearing tightness X, to obtain the real body temperature T and the wearing tightness X. For a specific calculation process, refer to the description in the foregoing embodiment, and details are not described herein again. In addition, the wearing behavior habit of the user can be evaluated based on the wearing tightness X, and guidance and correction are performed on the wearing behavior habit. It should be noted that the guidance or correction process may be implemented by using, but is not limited to, voice broadcast of the earphone, or may be displayed by using an interface of the external electronic device 6 connected to the earphone.

In this embodiment of this application, the first temperature sensor 4, the second temperature sensor 7, the third temperature sensor 8, and the fourth temperature sensor 10 are all disposed in the earphone, to measure the temperature of the periosteum by using the first temperature sensor 4, measure the human ear skin temperature by using the second temperature sensor 7, measure the ambient temperature by using the third temperature sensor 8, and measure the temperature of the heat emitting element 9 by using the fourth temperature sensor 10. Therefore, the ambient temperature measured by the third temperature sensor 8 is corrected based on the temperature measured by the fourth temperature sensor 10, to obtain a corrected ambient temperature, and therefore the obtained ambient temperature is more accurate. Then, according to different degrees of impact of the wearing tightness of the earphone and the ambient temperature on the first temperature sensor 4 and the second temperature sensor 7, a body temperature value of the human body is obtained based on a function relationship between the temperature measured by the first temperature sensor 4, the wearing tightness of the earphone, the ambient temperature, and the body temperature of the human body, and a function relationship between the temperature measured by the second temperature sensor 7, the wearing tightness of the earphone, the ambient temperature, and the body temperature of the human body, to eliminate impact of the wearing tightness and the ambient temperature and the temperature of the heat emitting element 9 in the earphone on the body temperature of the human body. In this way, a requirement of earphone temperature measurement on the wearing tightness of the earphone and impact of the ambient temperature and the temperature of the heat emitting element 9 on earphone temperature measurement are eliminated, to implement accurate temperature measurement in a comfortable and natural wearing status of the user, at various ambient temperatures, and in different application scenarios.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1.  An earphone, comprising an earbud and a first temperature sensor, wherein

    the earbud comprises an earbud housing and a sound outlet hole, the earbud housing has first accommodating space, the sound outlet hole is disposed on the earbud housing, and the sound outlet hole connects the first accommodating space and external space of the earphone; and
    the first temperature sensor is located in the first accommodating space, the first temperature sensor is disposed on an outer side of a hole channel of the sound outlet hole, and the first temperature sensor is in thermally conductive contact with the sound outlet hole through a sound outlet net.

2.  The earphone according to claim 1, wherein at least a part of the sound outlet net is provided with a thermally conductive path, and the first temperature sensor is in thermally conductive contact with the thermally conductive path.

3.  The earphone according to claim 2, wherein a material of the thermally conductive path is copper, stainless steel, nickel, titanium, gold, or silver.

4.  The earphone according to any one of claims 1 to 3, wherein the earphone further comprises a circuit board, the first temperature sensor is electrically connected to the circuit board, the circuit board is disposed on the outer side of the hole channel of the sound outlet hole, and the circuit board is in thermally conductive contact with the sound outlet hole.

5. The earphone according to any one of claims 1 to 4, wherein the earphone further comprises a second temperature sensor, the second temperature sensor is located in the first accommodating space, and the second temperature sensor is fastened to an inner side wall corresponding to a position at which the earbud housing is in contact with human ear skin.

6. The earphone according to claim 5, wherein the earbud housing is provided with a first thermally conductive portion, the first thermally conductive portion is made of a metal material, the first thermally conductive portion is in contact with the human ear skin, and the second temperature sensor is in thermally conductive contact with the first thermally conductive portion.

7. The earphone according to any one of claims 1 to 6, wherein the earphone further comprises an earphone handle, the earphone handle is fastened to the earbud, the earphone handle comprises an earphone handle housing, and the earphone handle housing has second accommodating space.

8. The earphone according to claim 7, wherein the earphone further comprises a third temperature sensor, the third temperature sensor is located in the second accommodating space, and the third temperature sensor is fastened to an inner side wall corresponding to a position at which the earphone handle housing is in contact with the external space.

9. The earphone according to claim 8, wherein the earphone handle housing is provided with a second thermally conductive portion, the second thermally conductive portion is made of a metal material, the second thermally conductive portion is in contact with the external space, and the third temperature sensor is in thermally conductive contact with the second thermally conductive portion.

10. The earphone according to claim 8 or 9, wherein the earphone further comprises a heat emitting element and a fourth temperature sensor, the heat emitting element is disposed in the first accommodating space and/or the second accommodating space, the fourth temperature sensor is disposed in the first accommodating space and/or the second accommodating space, and the fourth temperature sensor is disposed close to the heat emitting element.

11. The earphone according to claim 10, wherein the fourth temperature sensor and the heat emitting element are disposed on two sides of a same circuit board, and the fourth temperature sensor is disposed facing away from the heat emitting element.

12. The earphone according to claim 10 or 11, wherein the fourth temperature sensor and the third temperature sensor are spaced from each other.

13. The earphone according to any one of claims 1 to 12, wherein the earphone further comprises a speaker, the speaker is accommodated in the first accommodating space, the speaker comprises a first sound emitting unit and a second sound emitting unit, and the second sound emitting unit is disposed between the first sound emitting unit and the sound outlet hole; and
the first sound emitting unit comprises a first diaphragm and a coil disposed on the first diaphragm, and the second sound emitting unit comprises a second diaphragm and a magnetic circuit component.

14. A temperature measurement method for an earphone, wherein the earphone comprises an earbud and a first temperature sensor, the earbud comprises an earbud housing and a sound outlet hole, the earbud housing has first accommodating space, the sound outlet hole is disposed on the earbud housing, and the sound outlet hole connects the first accommodating space and external space of the earphone; the first temperature sensor is located in the first accommodating space, the first temperature sensor is disposed on an outer side of a hole channel of the sound outlet hole, and the first temperature sensor is in thermally conductive contact with the sound outlet hole; and the method comprises:

performing wear detection on the earphone, and determining a wearing status of the earphone on a human ear; triggering a temperature measurement procedure of the earphone based on the wearing status of the earphone on the human ear, and controlling the first temperature sensor to start to collect temperature data; and processing the temperature data collected by the first temperature sensor to obtain a human body temperature.

15. The method according to claim 14, wherein the processing the temperature data collected by the first temperature sensor to obtain a human body temperature specifically comprises:

forming a temperature data curve based on the temperature data collected by the first temperature sensor; and processing the temperature data curve to obtain the human body temperature.

**16.** The method according to claim 14, wherein the earphone further comprises a second temperature sensor, the second temperature sensor is configured to measure a human ear skin temperature, and the processing the temperature data collected by the first temperature sensor to obtain a human body temperature specifically comprises: obtaining the human body temperature based on the temperature data collected by the first temperature sensor and temperature data collected by the second temperature sensor.

**17.** The method according to claim 16, wherein the earphone further comprises a third temperature sensor, the third temperature sensor is configured to measure an ambient temperature of the external space of the earphone, and the method further comprises:
obtaining the human body temperature based on the temperature data collected by the first temperature sensor, the temperature data collected by the second temperature sensor, and temperature data collected by the third temperature sensor.

**18.** The method according to claim 17, wherein the earphone further comprises a heat emitting element and a fourth temperature sensor, the fourth temperature sensor is configured to measure a temperature of the heat emitting element, and the method further comprises:
obtaining the human body temperature based on the temperature data collected by the first temperature sensor, the temperature data collected by the second temperature sensor, the temperature data collected by the third temperature sensor, and temperature data collected by the fourth temperature sensor.

**19.** The method according to claim 18, wherein the method further comprises:

correcting, based on the temperature data collected by the fourth temperature sensor, the temperature data collected by the third temperature sensor, and obtaining a corrected ambient temperature; and
obtaining the human body temperature based on the temperature data collected by the first temperature sensor, the temperature data collected by the second temperature sensor, and the corrected ambient temperature.

**20.** The method according to any one of claims 14 to 19, wherein the earphone is connected to an external electronic device, the external electronic device has a display interface, and the method further comprises:
displaying the obtained human body temperature on the display interface.

FIG. 1

FIG. 2

FIG. 3

1021

102

1

1011

4

5

FIG. 4

| Perform wear detection |

↓

| Trigger a temperature measurement procedure of an earphone |

↓

| Record temperature data collected by a first temperature sensor within a specified time period |

↓

| Process the temperature data to obtain a human body temperature |

FIG. 5

6

FIG. 6

FIG. 7a

FIG. 7b

FIG. 7c

Temperature °C

40
38
36
34
32
30
28
26
24

Temperature change status

Time s

FIG. 8

1021

4

7

101(1)

5

1011

FIG. 9

FIG. 10

Temperature transfer direction

Heat area

Ambient temperature

TD                                    TC

FIG. 11

FIG. 12

## INTERNATIONAL SEARCH REPORT

| International application No. |
|---|
| **PCT/CN2022/111679** |

| A. | CLASSIFICATION OF SUBJECT MATTER |
|---|---|
| | H04R 1/10(2006.01)i |

According to International Patent Classification (IPC) or to both national classification and IPC

| B. | FIELDS SEARCHED |
|---|---|

Minimum documentation searched (classification system followed by classification symbols)

H04R1/-

Documentation searched other than minimum documentation to the extent that such documents are included in the fields searched

Electronic data base consulted during the international search (name of data base and, where practicable, search terms used)

CNABS, CNTXT, ENTXTC, CNKI: 耳机, 耳温, 温度, 体温, 热, 耳膜, 骨膜, 传递, 传导, 导热, 出音, 出声, 遮挡, 变小, 变窄, 防尘, 网, 传感器, 感测, 感应, 探测, 热敏, 红外; VEN, ENTXT, USTXT, EPTXT, WOTXT: Earpiece, Earphone, temperature, heat, eardrum, transmit+, conduct+, sound, shield, reduce, narrow, dust, mesh, sens+, detect+, infrared

| C. | DOCUMENTS CONSIDERED TO BE RELEVANT | |
|---|---|---|
| Category* | Citation of document, with indication, where appropriate, of the relevant passages | Relevant to claim No. |
| A | CN 210327920 U (CHENGDU BISHENG TECHNOLOGY CO., LTD.) 14 April 2020 (2020-04-14)<br>entire document | 1-20 |
| A | CN 213213815 U (ZHENGZHOU UNIVERSITY) 14 May 2021 (2021-05-14)<br>entire document | 1-20 |
| A | CN 210298005 U (SHENZHEN ZHONGNUO COMMUNICATION CO., LTD.) 10 April 2020 (2020-04-10)<br>entire document | 1-20 |
| A | JP 2002224050 A (ISUZU MOTORS LTD.) 13 August 2002 (2002-08-13)<br>entire document | 1-20 |
| A | US 2021267546 A1 (CHEN, Kaichun) 02 September 2021 (2021-09-02)<br>entire document | 1-20 |
| A | US 2018368718 A1 (KOZIOL, Jeffrey E.) 27 December 2018 (2018-12-27)<br>entire document | 1-20 |

| ✓ Further documents are listed in the continuation of Box C. | ✓ See patent family annex. |
|---|---|

| | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 September 2022** | **16 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/111679**

**C.    DOCUMENTS CONSIDERED TO BE RELEVANT**

| Category* | Citation of document, with indication, where appropriate, of the relevant passages | Relevant to claim No. |
|---|---|---|
| A | 杨阳等 (YANG, yang et al.). "一种监测人体体温及脉搏波的耳机装置 (An earphone device for monitoring body temperature and pulse waves of a human body)" <br> 中国医学装备 (China Medical Equipment), Vol. 5, No. 10, 15 May 2013 (2013-05-15), entire document | 1-20 |

Form PCT/ISA/210 (second sheet) (January 2015)

## INTERNATIONAL SEARCH REPORT
### Information on patent family members

International application No.

**PCT/CN2022/111679**

| Patent document cited in search report | | | Publication date (day/month/year) | Patent family member(s) | | | Publication date (day/month/year) |
|---|---|---|---|---|---|---|---|
| CN | 210327920 | U | 14 April 2020 | None | | | |
| CN | 213213815 | U | 14 May 2021 | None | | | |
| CN | 210298005 | U | 10 April 2020 | None | | | |
| JP | 2002224050 | A | 13 August 2002 | None | | | |
| US | 2021267546 | A1 | 02 September 2021 | TW | M596600 | U | 11 June 2020 |
| US | 2018368718 | A1 | 27 December 2018 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111233939